# EUROPEAN PATENT APPLICATION

(11) **EP 4 755 338 A1**
(43) Date of publication of application: **10.06.2026**
(21) Application number: 24307043.0
(22) Date of filing: 05.12.2024
(51) Int. Cl.: A61B 34/30, A61B 90/00, A61B 90/50, B25J 5/00, B25J 5/02, A61B 1/227, A61B 1/233, A61B 1/24

(54) **DEVICE FOR POSITIONING A PLATFORM**

(71) Applicant: Zentact Robotics, 38400 Saint Martin d'Hères (FR)
(72) Inventor: ARBARET, Valentin, 38400 Saint Martin d'Hères (FR); PHILBERT-ROUTIER, Damien, 38400 Saint Martin d'Hères (FR)
(74) Representative: Regimbeau

(57) **Abstract**

The present disclosure relates to a device for positioning a platform (200) according to six degrees of freedom, comprising:
- a base (100);
- a platform (200);
- six actuators (600) pivotally attached to the base (100) and to the platform (200);
- a seventh actuator configured to translate the base (100) along a longitudinal direction (L); and
- a guiding support (300) comprising a mechanical tilting system configured to tilt the base (100) around a tilt axis in response to a translation of the base (100) along the longitudinal direction (L) so that an actuation of the seventh actuator results in a combined translation of the base (100) along the longitudinal direction (L) and rotation of the base (100) around the tilt axis.

## Description

### TECHNICAL FIELD

The present disclosure relates to the field of devices for positioning a platform according to six degrees of freedom, especially in the context of otorhinolaryngology surgery.

### TECHNICAL BACKGROUND

In the current practice, surgeons such as otorhinolaryngology surgeons often need to manually position an endoscope to visualize the area of intervention while simultaneously operating surgical instruments with both hands. This manual approach poses significant ergonomic challenges, as the surgeon must continuously adjust the endoscope's position to maintain optimal visibility while performing precise movements with other surgical instruments. This increases complexity and workload of the surgical procedure for the surgeon, limiting the smoothness of movements and overall efficiency of the procedure.

Stewart platforms, also known as hexapods, are commonly used in applications requiring high precision, such as flight simulators. Stewart platforms allow precise positioning of an object across six degrees of freedom through a mobile platform connected to a fixed base by six linear actuators. Document US 11 612 438 B2 discloses a device using a Stewart platform for a medical operation, a surgical tool being mounted on the Stewart platform. However, the device needs to be handheld. Moreover, Stewart platforms are bulky, thus their size is not well-suited to the confined spaces of surgical environments. In addition, the actuators in Stewart platforms have limited stroke lengths, which restricts the range of motion for positioning the platform and thus reduces flexibility and effectiveness during procedures such as surgical procedures. Additionally, the movement of the actuators is relatively slow, which increases the time needed to adjust the Stewart platform's position or store the Stewart platform after use, thereby extending the overall duration for example of the surgical procedure.

### SUMMARY

An aim of the present disclosure is to propose a device for positioning a platform according to six degrees of freedom which is compact and easy to use.

Another aim of the present disclosure is to propose a device capable of quickly positioning a platform with six degrees of freedom over a wide range of positions.

According to a first aspect, the present disclosure relates to a device for positioning a platform according to six degrees of freedom, comprising:
- a base;
- a platform;
- six actuators, each comprising a first end pivotally attached to the base and a second end pivotally attached to the platform, the six actuators being configured to be individually or simultaneously actuated so as to rotate and translate the platform relative to the base according to the six degrees of freedom;
- a seventh actuator configured to translate the base along a longitudinal direction; and
- a guiding support comprising a mounting system for a movable attachment of the base in translation along the longitudinal direction and in tilting around a tilt axis perpendicular to the longitudinal direction, wherein the guiding support further comprises a mechanical tilting system cooperating with the base and configured to tilt the base around the tilt axis in response to a translation of the base along the longitudinal direction so that an actuation of the seventh actuator results in a combined translation of the base along the longitudinal direction and rotation of the base around the tilt axis.

The mechanical tilting system may be configured to maintain a tilt of the base equal to zero when a stroke of the seventh actuator is below a predetermined threshold, and to increase a tilt of the base when the stroke of the seventh actuator is above the predetermined threshold.

The mechanical tilting system may comprise a rib having a flat portion extending mainly along the longitudinal direction and a curved portion. The mechanical tilting system further comprises an articulated joint mounting the base on the rib. The curved portion may extend from the flat portion.

The curved portion may have a fixed radius of curvature.

The curved portion may comprise a first end and a second end opposite to the first end along the longitudinal direction, wherein the first end of the curved portion is a straight edge and the second end of the curved portion is a straight edge, and wherein a first plane tangent to the curved portion at a first point located on the first end is substantially perpendicular to a second plane tangent to the curved portion at a second point located on the second end.

The mounting system of the guiding support may comprise at least one linear longitudinal guide rail and at least one sliding joint, each sliding joint mounting the base on a respective guide rail.

A central bore may be formed in the base, the rib or the guide rail being configured to extend facing the central bore.

The six actuators may comprise hydraulic and/or electric cylinders.

The seventh actuator may be a hydraulic or an electric cylinder.

The guiding support may comprise a fixing system configured for removable attachment of the guiding support to a component located in a surgical space.

The platform may comprise a fastening system configured for removable attachment of a surgical tool to the platform.

According to a second aspect, the present disclosure relates to a surgical apparatus comprising a device according to the first aspect and a control unit configured to:
- receive a target position of the platform;
- calculate respective movement orders for the six actuators and the seventh actuator in order to move the platform to the target position; and
- send the respective movement orders to the respective six actuators and seventh actuator so as to correspondingly move the six actuators and seventh actuator in order to reach the target position of the platform.

The surgical apparatus may further comprise a surgical tool in the form of an endoscope or a videoscope configured to be removably attached to the platform by the fastening system of the device.

The control unit may be configured to calculate the movement orders continuously and in real time and to send the movement orders continuously and in real time, simultaneously to the six actuators and to the seventh actuator.

According to a third aspect, the present disclosure relates to a method for positioning a platform of a device according to the first aspect, comprising the following steps:
- receiving a target position of the platform;
- calculating respective movement orders for the six actuators and the seventh actuator in order to move the platform to the target position; and
- sending the respective movement orders to the respective six actuators and seventh actuator so as to correspondingly move the six actuators and seventh actuator in order to reach the target position of the platform.

### BRIEF DESCRIPTION OF THE DRAWINGS

Other features and advantages of the disclosure will appear in the following description with reference to the drawings, in which:
Figure 1 illustrates a schematic perspective view of a device for positioning a platform according to a first embodiment, the device being in a nominal configuration.
Figure 2 illustrates a schematic side view of the device of figure 1.
Figure 3 illustrates a schematic perspective view of the device of figure 1, the device being in a threshold configuration.
Figure 4 illustrates a schematic side view of the device of figure 3.
Figure 5 illustrates a schematic perspective view of the device of figure 1, the device being in a tilted configuration.
Figure 6 illustrates a schematic side view of the device of figure 5.
Figure 7 illustrates a schematic perspective view of the device of figure 1 used in a first surgical procedure.
Figure 8 illustrates a schematic perspective view of the device of figure 1 used in a second surgical procedure.
Figure 9 illustrates a schematic kinematic view of the device of figure 1.
Figure 10 illustrates a schematic perspective view of a device for positioning a platform according to a second embodiment, the device being in a nominal configuration.

### DETAILED DESCRIPTION

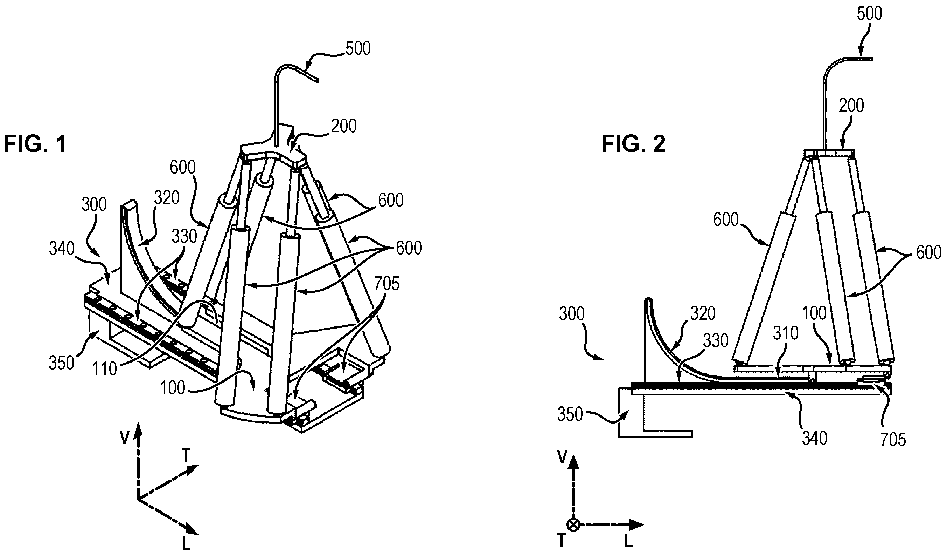

A device for positioning a platform 200 according to six degrees of freedom is illustrated by way of a non-limiting example in figure 1. The device comprises:
- a base 100;
- a platform 200;
- six actuators 600, each comprising a first end pivotally attached to the base 100 and a second end pivotally attached to the platform 200, the six actuators 600 being configured to be individually or simultaneously actuated so as to rotate and translate the platform 200 relative to the base 100 according to the six degrees of freedom;
- a seventh actuator configured to translate the base 100 along a longitudinal direction L; and
- a guiding support 300 comprising a mounting system for a movable attachment of the base 100 in translation along the longitudinal direction L and in tilting around a tilt axis perpendicular to the longitudinal direction L, wherein the guiding support 300 further comprises a mechanical tilting system cooperating with the base 100 and configured to tilt the base 100 around the tilt axis in response to a translation of the base 100 along the longitudinal direction L so that an actuation of the seventh actuator results in a combined translation of the base 100 along the longitudinal direction L and rotation of the base 100 around the tilt axis.

It should be understood that the term "positioning" refers to setting the platform's 200 location and orientation. The term "positioning" thus covers all aspects of the platform's 200 movement, including translations and rotations, the six degrees of freedom including three degrees of freedom in translation and three degrees of freedom in rotation.

The six actuators 600 pivotally attached to the base 100 and the platform 200 allow moving the platform 200 in any translational direction and/or rotate the platform 200 around any axis of rotation, within the range of accessible positions of the device. Thus, the disclosed six actuators 600 enable a precise and complex positioning of the platform 200, thus of a surgical tool 500 mounted on the platform 200, along the six degrees of freedom.

Furthermore, the device can position the platform 200 in a wide range of positions, thus provides an extended work area. Therefore, the device can be used in a wide variety of applications, more specifically in varied surgical procedures. For example, in otorhinolaryngology procedures, such a device allows to easily position a surgical tool 500 fixed to the platform 200 at the nose 810, the ear 820 or the larynx of a patient 800, for different positions of the patient 800 on an operation table.

Additionally, the seventh actuator and the mechanical tilting system make it possible to tilt the base 100 by a separated mechanism, therefore to more quickly and efficiently position the platform 200 in a wider range of positions. For example, during a surgical procedure, the surgical tool 500 mounted on the platform 200 can be quickly positioned in the desired position, and at the end of the surgical procedure the device can be quickly stored.

Finally, by combining a translation and a tilt of the base 100, the mechanical tilting system allows improving a compacity of the device for a given range of accessible positions of the platform 200. Thus, the surgeon can easily position the device in front of him and place his/her hands on each side of the device in order to manipulate his/her surgical tools during the surgical procedure.

### Six actuators

The six actuators 600 are configured to be individually or simultaneously actuated so as to rotate and translate the platform 200 relative to the base 100 according to the six degrees of freedom. In other words, one, several or all of the six actuators 600 may be actuated simultaneously in order to move the base 100 to a given position, each of the six actuators 600 being able to extend or retract independently from the other five actuators 600. The combined action of one, several or all of the six actuators 600 allows precise control over the position, that is to say over the location and orientation, of the platform 200. By controlling the extension and retraction of each of the six actuators 600 in precise amounts, the platform 200 can be moved in any direction and/or orientation simultaneously. This means that the platform 200 can translate in space while rotating, providing six degrees of freedom for complex motions.

The six actuators 600 may be linear actuators, that is to say that each of the six actuators 600 may extend or retract along a straight line, within a given stroke range of the actuator 600. The stroke ranges of the six actuators 600 may be identical to each other. The stroke ranges of the six actuators 600 may be chosen according to a desired range of accessible locations and orientations of the platform 200 relative to the base 100. Actuating a respective actuator 600 changes the stroke of said respective actuator 600.

The six actuators 600 may comprise electric actuators, hydraulic actuators and/or pneumatic actuators. In other words, among the six actuators 600, one or more may be electric actuators and/or one or more may be hydraulic actuators and/or one or may be more pneumatic actuators. For example, the six actuators 600 may be identical electric actuators.

### Base, platform

The base 100 may comprise a base plate on which the six actuators 600 are mounted. The platform 200 may comprise a platform plate on which the six actuators 600 are mounted.

The six actuators 600 may be attached to the base 100 and to the platform 200 in any configuration allowing movement of the platform 200 relative to the base 100 in the six degrees of freedom. Each of the six actuators 600 is pivotally fixed to the base 100, for example by a base pivot joint 601, for example by a spherical joint or a universal joint. Each of the six actuators 600 is pivotally fixed to the platform 200, for example by a platform pivot joint 602, for example by a spherical joint or a universal joint.

Three base pivot points may be arranged on the base 100, for example on the base plate. The three base pivot points may be substantially equidistant to one another and separated from a center of the base 100 by a same distance corresponding to a base radius. In the following, it is considered that each base pivot point is located at a vertex of the base 100. Two base pivot joints 601 of the base 100 may be located on each side of each respective base pivot point. For example, two base pivot joints 601 may be located symmetrically with respect to each respective base pivot point.

Three platform pivot points may be arranged on the platform 200, for example on the platform plate. The three platform pivot points may be substantially equidistant to one another and separated from a center of the base 100 by a same distance corresponding to a platform radius. In the following, it is considered that each platform pivot point is located at a vertex of the platform 200. Two platform pivot joints 602 may be located on each side of each respective platform pivot point. For example, two platform pivot joints 602 may be located symmetrically with respect to each respective platform pivot point.

The six actuators 600 are thus distributed around the platform 200 and the base 100. The first radius of the base 100 may be greater than the second radius of the platform 200.

In a nominal configuration of the device, as illustrated by way of a non-limiting example in figure 1, corresponding to nominal strokes of each of the six actuators 600 and the seventh actuator, the base 100 and the platform 200 may extend substantially parallel to each other. The tilt of the base 100 may be equal to 0°. The vertices of the base 100 and the vertices of the platform 200 may be arranged facing each other or alternatively may be arranged in a staggered alignment, each vertex of the base 100 being located between two vertices of the platform 200, and conversely each vertex of the platform 200 being located between two vertices of the base 100.

A central bore 110 may be formed in the base 100, more specifically may be formed in the base plate. The central bore 110 extends along the longitudinal direction L in the nominal configuration. The central bore 110 may extend forward from a back end of the base 100 or may extend backward from a front end of the base 100.

The platform 200 may comprise a fastening system configured for removable attachment of a surgical tool 500 to the platform 200. Thus, the surgical tool 500 can easily be mounted on the platform 200 and/or removed from the platform 200, for example to be replaced by another surgical tool 500, according to the needs of the surgeon.

The fastening system may comprise any system configured to removably attach the surgical tool 500 to the platform 200, for example may comprise clips, magnets, one or more screws and bolts, etc.

A vertical direction V corresponds to a direction perpendicular to the base 100 and to the platform 200 in the nominal configuration, the vertical direction V being perpendicular to the longitudinal direction L. In the following, the terms lower than, or under, are used in reference to a position along the vertical direction V, the base 100 being located under the platform 200 in the nominal configuration.

A lateral direction T corresponds to a direction perpendicular to the vertical direction V and the longitudinal direction L. The lateral direction T may correspond to the direction of the tilt axis.

Dimensions of the base plate may be strictly greater than dimensions of the platform plate. Distances separating two vertices of the base 100 may be strictly greater than distances separating two vertices of the platform 200. Thus, in the nominal configuration, the six actuators 600 extend between the base 100 and platform 200, at a non-zero angle relative the vertical direction V. The angles formed between each of the six actuators 600 and the vertical direction V may be substantially identical to each other. The angles formed between each of the six actuators 600 and the vertical direction V may be adjusted to provide a suitable stability of the platform 200. Each of the six actuators 600 may extend in different directions.

### Seventh actuator

The seventh actuator (not illustrated on the figures) is configured to be individually or simultaneously actuated with the six actuators 600.

The seventh actuator may be a linear actuator, that is to say it may extend or retract along a straight line, within a given stroke range of the seventh actuator. The seventh actuator is thus actuated between a minimum stroke and a maximum stroke of the seventh actuator. The stroke range of the seventh actuator may be chosen according to dimensions of the mounting system of the guiding support 300, a desired range of position of the base 100 along the longitudinal direction L and/or a desired range of tilting of the base 100 relative to the tilt axis. Actuating the seventh actuator changes the stroke of the seventh actuator.

The seventh actuator may be an electric actuator, a hydraulic actuator or a pneumatic actuator.

The seventh actuator may be attached to the base 100 by a joint 705 such as a fixed joint, a sliding joint and/or by a pivot joint, for example by a spherical joint or a universal joint. The joint 705 attaching the seventh actuator to the base 100 may be located under the base plate.

In the nominal configuration of the device, the seventh actuator may extend along a longitudinal axis parallel to or coinciding with the longitudinal direction L and the stroke of the seventh actuator may correspond to the minimum stroke of the seventh actuator. The seventh actuator may extend along the longitudinal direction L in all of the accessible configurations of the device.

The device may comprise an eighth actuator (not illustrated on the figures). The eighth actuator may be substantially identical to the seventh actuator. The eighth actuator is attached to the base 100 parallel to the seventh actuator. Simultaneous and identical actuations of the seventh actuator and of the eighth actuator are used to translate the base 100 along the longitudinal direction L.

In the following, the terms forward and backward, or front and behind, are used in reference to a position along the longitudinal direction L, the joint 705 attaching the seventh actuator to the base 100 forming a back end of the seventh actuator.

### Guiding support

The guiding support 300 may comprise a plate 340 substantially perpendicular to the vertical direction V. The mounting system and the mechanical tilting system may be mounted on the guiding support plate 340.

### Mounting system

The mounting system of the guiding support 300 attaching the base 100 to the guiding support 300 may comprise at least one articulated joint allowing both a translation of the base along the longitudinal direction L and a tilting of the base around the tilt axis. For example, the mounting system may comprise at least one sliding joint 335 directed along the longitudinal direction L and at least one pivot joint 336 directed along the tilt axis, each pivot joint 336 being for example mounted between each sliding joint 335 and the base 100.

When the articulated joint of the mounting system is translated along the longitudinal direction L, the platform 200 is correspondingly translated along the longitudinal direction L. When the articulated joint of the mounting system is translated along the vertical direction V, the platform 200 is rotated around the tilt axis. Thus, the combined movement of translation along the longitudinal direction L and along the vertical direction V of the articulated joint of the mounting system results in a combined translation of the platform 200 along the longitudinal direction L and rotation of the platform 200 around the tilt axis.

The mounting system of the guiding support 300 may comprise at least one linear longitudinal guide rail 330 and at least one sliding joint 335, each sliding joint 335 mounting the base 100 on a respective guide rail 330. The guide rail 330 extends along the longitudinal direction L between a front end and a rear end. Such a guide rail 330 allows a precise and easy to implement guiding of the base 100 in translation along the longitudinal direction L, an actuation of the seventh actuator generating a corresponding translation of the base 100 along the guide rail 330, thus along the longitudinal direction L.

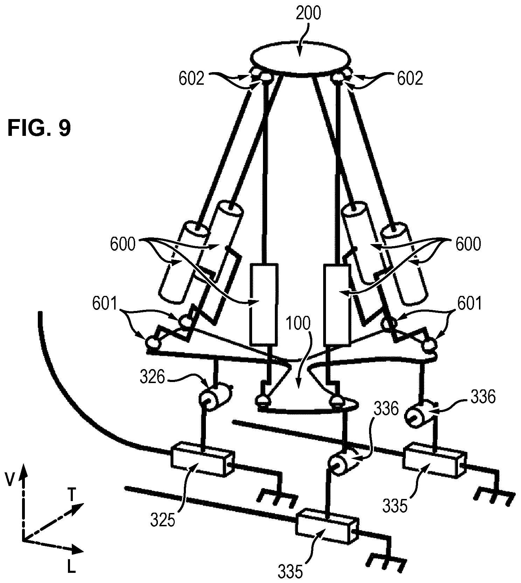

The mounting system may also comprise at least one pivot joint 336, each pivot joint 336 being mounted between a corresponding sliding joint 335 and the base 100, in order to allow tilting of the base 100. The sliding joint 335 and the corresponding pivot joint 336 may be located under the base plate. Figure 9 illustrates an example of such pivot joints 336.

The tilt axis may correspond to an axis along which the pivot joint 336 is directed. It is noted that while a direction of the tilt axis is fixed, and may for example correspond to the lateral direction T, a position of the tilt axis varies as the tilt axis translates with the base along the longitudinal direction L.

The guide rail 330 may be mounted directly on the guiding support plate 340. A length of the guide rail 330 may be equal or superior to the stroke range of the seventh actuator.

A dimension of the central bore 110 of the base 100 in the lateral direction T may correspond substantially to a dimension of the guide rail 330 in the lateral direction T. The guide rail 330 may be configured to extend facing the central bore 110. More specifically, a part of the guide rail 330 may be configured to extend right below the central bore 110 of the base 100.

In the nominal configuration, that is to say for the minimum stroke of the seventh actuator, the sliding joint 335 mounting the base 100 on the guide rail 330 may be located at the front end of the guide rail 330, as illustrated by way of a non-limiting example in figure 2. For the maximum stroke of the seventh actuator, the sliding joint 335 mounting the base 100 on the guide rail 330 may be located between the front end and the rear end of the guide rail 330, or at the rear end of the guide rail 330.

### Mechanical tilting system

The mechanical tilting system may comprise any system capable of mechanically correlating a translation of the base 100 along the longitudinal direction L with a tilt of the base 100 around the tilt axis. The mechanical tilting system allows for managing a larger number of base 100 positions using a simple and reliable system with only slightly increased mass and size.

The mechanical tilting system may be configured to increase the tilt of the base 100 in any conceivable way, for example regularly or irregularly, as the stroke of the seventh actuator is increased, that is to say as the base 100 is translated backward along the longitudinal direction L.

The mechanical tilting system may be configured to maintain a tilt of the base 100 equal to zero when a stroke of the seventh actuator is below a predetermined threshold, and to increase a tilt of the base 100 when the stroke of the seventh actuator is above the predetermined threshold.

For the minimum stroke of the seventh actuator, the tilt of the base 100 is equal to zero. For the maximum stroke of the seventh actuator, the tilt of the base 100 is maximum and strictly greater than zero. The maximum tilt of the base 100 may be greater than 45°, for example may be equal to 90°.

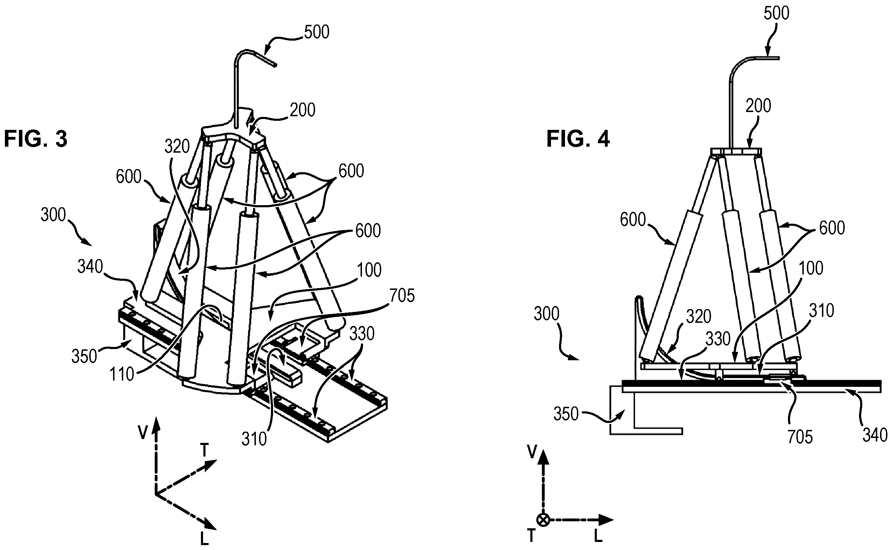

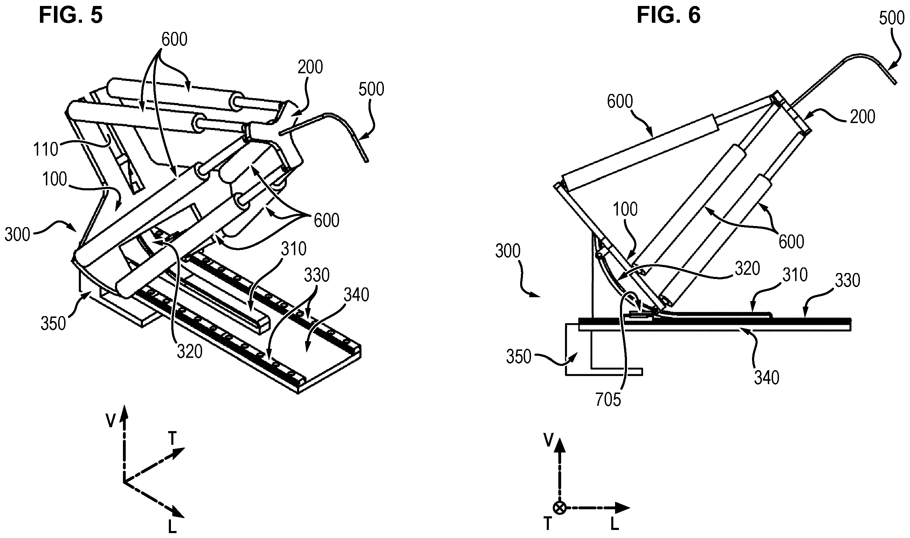

The predetermined threshold may be strictly superior to the minimum stroke of the seventh actuator and strictly inferior to the maximum stroke of the seventh actuator. Thus, an actuation of the seventh actuator between the minimum stroke and the predetermined threshold results in a pure translation of the base 100 along the longitudinal direction L, while an actuation of the seventh actuator between the predetermined threshold and the maximum stroke results in a combined translation of the base 100 along the longitudinal direction L and tilting of the base 100 around the tilt axis. Figure 3 and figure 4 illustrate a threshold configuration where the stroke of the seventh actuator is equal to the predetermined threshold and the base 100 is not tilted. Figure 5 and figure 6 illustrate a tilted configuration where the stroke of the seventh actuator is greater than the predetermined threshold and the base 100 is tilted by an angle strictly greater than zero.

Alternatively, the predetermined threshold may be equal to the minimum stroke of the seventh actuator, an actuation of the seventh actuator always resulting in a combined translation of the base 100 along the longitudinal direction L and tilting of the base 100 around the tilt axis.

The mechanical tilting system may comprise a rib having a flat portion 310 extending mainly along the longitudinal direction L, and an additional portion having a non-zero inclination relative to the longitudinal direction L. Thus, the additional portion guides a tilt of the base 100 around the tilt axis. The additional portion may have any shape suitable for allowing a combined translation of the base 100 along the longitudinal direction L and tilting of the base 100 around the tilt axis.

The additional portion may thus comprise at least one flat oblique portion forming a non-zero angle relative to the longitudinal direction L. For example, the additional portion may comprise several additional flat oblique portions forming progressively steeper angles relative to the longitudinal direction L. This way, as the base 100 is moved along the additional portion, the tilt of the base 100 around the tilt axis is increased.

Alternatively, the mechanical tilting system may comprise a rib having a flat portion 310 extending mainly along the longitudinal direction L and a curved portion 320. The mechanical tilting system further comprises an articulated joint mounting the base 100 on the rib. The flat portion 310 allows translating the base 100 along the longitudinal direction L, while the curved portion 320 allows combined translation of the base 100 along the longitudinal direction L and tilting of the base 100 around the tilt axis. Such a mechanical tilting system is easy to implement and allows to mechanically tilt the base 100 relative to the tilt axis when the articulated joint is positioned facing the curved portion 320, the displacement of the base 100 being then guided by the curved portion 320, while allowing a pure translation of the base 100 along the longitudinal direction L when the articulated joint is positioned facing the flat portion 310, the displacement of the base 100 being then guided by the flat portion 310.

The curved portion 320 may extend from the longitudinal flat portion 310 or from another portion of the rib, for example from another flat portion arranged at an angle from the longitudinal flat portion 310.

The rib may also comprise one or more oblique portion(s), for example extending from the curved portion 320. The oblique portion extending from the curved portion 320 forms an angle relative to the longitudinal direction L, which may correspond to an angle formed between the longitudinal direction L and a tangent to the curved portion 320 at a point of connection with the oblique portion.

In the nominal configuration of the device, that is to say for the minimum stroke of the seventh actuator, the articulated joint may be positioned facing the flat portion 310. The articulated joint may remain positioned facing the flat portion 310 when the stroke of the seventh actuator is comprised between the minimum stroke and the predetermined threshold. The articulated joint may be positioned facing the curved portion 320 when the stroke of the seventh actuator is comprised between the predetermined threshold and the maximum stroke.

The flat portion 310 of the mechanical tilting system extends substantially parallel to the guide rail 330. The flat portion 310 may be mounted directly on the guiding support plate 340. The flat portion 310 may comprise a front end and a back end opposite the front end in the longitudinal direction L.

The articulated joint may mount a back end of the base 100 on the rib while the sliding joint 335 mounts a front end of the base 100 on the guide rail 330. In the nominal configuration, the articulated joint mounting the base 100 on the rib extends behind the sliding joint 335 mounting the base 100 on the guide rail 330. The front end of the flat portion 310 may extend strictly behind the front end of the guide rail 330. When the stroke of the seventh actuator is above the predetermined threshold, the base 100 is tilted such that the back end of the base 100 is located above the front end of the base 100.

The articulated joint may comprise a curved translation joint 325 allowing a translation of the base 100 along the curved portion 320, and a pivot joint 326 allowing a tilt of the base 100 around the tilt axis, as illustrated by way of a non-limiting example in figure 9. The curved translation joint 325 may be mounted on the rib, while the pivot joint 326 is mounted between the curved translation joint 325 and the base 100. The pivot joint 326 may be directed along the tilt axis, the pivot joint 326 enabling a rotation around the tilt axis.

The curved portion 320 of the mechanical tilting system may extend substantially in the longitudinal direction L. The curved portion 320 may be mounted directly on the guiding support plate 340 and extend above the guiding support plate 340 in the longitudinal direction L and the vertical direction V. The curved portion 320 may comprise a first end and a second end longitudinally opposite to the first end. The first end of the curved portion 320 may form a straight edge and the second end of the curved portion 320 may form a straight edge. The first end of the curved portion 320 may coincide with the back end of the flat portion 310, the curved portion 320 extending behind the flat portion 310. The second end of the curved portion 320 may extend behind and above the first end of the flat portion 310.

The curved portion 320 may have any shape allowing to tilt the base 100 as the base 100 is translated backwards in the longitudinal direction L. The shape of the curved portion 320 may be chosen according to the desired coupling between translation and tilt of the base 100. The curved portion 320 may have a concave shape.

The curved portion 320 may have a fixed radius of curvature. Such a fixed radius of curvature is easy to manufacture, mechanically efficient to guide the tilting of the base 100 and makes the positions of the platform 200 easy to compute. Alternatively, the curved portion 320 may have a variable radius of curvature.

The curved portion 320 may span over an arc, possibly with a variable radius of curvature. For example, the curved portion 320 may span over an arc having an angular range greater than 45°. The greater the arc over which the curved portion 320 spans, the greater the accessible tilt of the base 100 relative to the platform 200, the greater the range of positioning of the platform 200 relative to the base 100.

A first plane tangent to the curved portion 320 at a first point located on the first end of the curved portion 320 may be substantially perpendicular to a second plane tangent to the curved portion 320 at a second point located on the second end of the curved portion 320, as illustrated by way of a non-limiting example in figure 4. In other words, the curved portion 320 may span over an arc having an angular range equal to 90°, while having various shapes and radii of curvature.

In the nominal configuration, that is to say for the minimum stroke of the seventh actuator, the articulated joint mounting the base 100 on the rib may be located at the front end of the flat portion 310, as illustrated by way of a non-limiting example in figure 2.

For the maximum stroke of the seventh actuator, the articulated joint mounting the base 100 on the rib may be located at the rear end of the curved portion 320. The articulated joint may for example be located behind and above, or directly above, the sliding joint 335 mounting the base 100 on the guide rail 330.

A dimension of the central bore 110 of the base 100 in the lateral direction T may correspond substantially to a dimension of the rib in the lateral direction T. The rib may be configured to extend facing the central bore 110. More specifically, the flat portion 310 of the rib may be configured to extend right below the central bore 110 of the base 100 while the additional portion of the rib such as the curved portion 320 of the rib may be configured to extend in the central bore 110 of the base 100 for given positions of the base 100 along the longitudinal direction L. Therefore, the central bore 110 allows tilting of the base 100 around the tilt axis without the base plate impeding the tilting of the base 100.

In a first embodiment, illustrated by way of a non-limiting example in figure 1, in figure 3 and in figure 5, the mounting system comprises two identical longitudinal guide rails 330 extending parallel to each other along the longitudinal direction L and the mechanical tilting system comprises one unique rib having a flat portion 310 and a curved portion 320. Each longitudinal guide rail 330 may be mounted on the base 100 by a respective sliding joint 335 and pivot joint 336. An axis connecting the two pivot joints 336 may correspond to the tilt axis. The two longitudinal guide rails 330 are separated from each other in the lateral direction T and extend on each side of the unique rib of the mechanical tilting system. The device also comprises an eighth actuator, each of the seventh actuator and the eighth actuator being mounted on a respective one of the two guide rails 330. Each longitudinal guide rail 330, thus each of the seventh actuator and the eighth actuator, may be mounted on a respective front vertex of the base 100. Each sliding joint 335 and corresponding pivot joint 336 are located at the respective front vertex of the base 100.

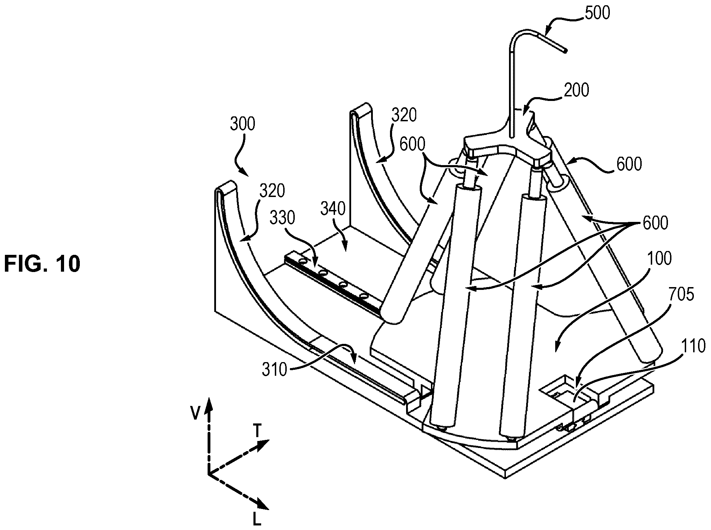

In a second embodiment, illustrated by way of a non-limiting example in figure 10, the mounting system comprises one unique longitudinal guide rail 330 and the mechanical tilting system comprises two identical ribs extending parallel to each other, each rib having a flat portion 310 and a curved portion 320. Each rib may be mounted on a respective front vertex of the base 100. The unique longitudinal guide rail 330 extends along the longitudinal direction L. The two ribs are separated from each other in the lateral direction T and extend on each side of the unique guide rail 330. The unique guide rail 330 is configured to extend facing the central bore 110. The joint 705 attaching the seventh actuator to the base 100 may extend facing the central bore 110.

### Fixing system

The guiding support 300 may comprise a fixing system 350 configured for removable attachment of the guiding support 300 to a component located in a surgical space. The component to which the guiding support may be fixed by the fixing system 350 may be a flat surface such as an operation table, or alternatively may be a robotic arm. Such a fixing system 350 allows the guiding support 300 to remain in a desired position relative to the patient 800, while the position of the platform 200 relative to the base 100 may be adapted. Moreover, the removable attachment allows to easily set in place or remove the device before or after the surgical operation.

The fixing system 350 may comprise any system configured to removably attach the guiding support 300 to the component, for example may comprise a clamp configured to tighten around an edge of a table, a suction cup configured to adhere to a flat surface, one or more screws and bolts configured to fasten the guiding support 300 to the component, etc.

The fixing system 350 may be fixed to the back end of the guiding support plate 340.

### Surgical apparatus, control unit

A surgical apparatus may comprise a device as disclosed above and a control unit configured to:
- receive a target position of the platform 200;
- calculate respective movement orders for the six actuators 600 and the seventh actuator in order to move the platform 200 to the target position; and
- send the respective movement orders to the respective six actuators 600 and seventh actuator so as to correspondingly move the six actuators 600 and seventh actuator in order to reach the target position of the platform 200.

The control unit determines how each of the six actuators 600 and the seventh actuator should move to achieve the desired motion of the platform 200 from a current position to the target position. The control unit calculates the required changes in the strokes of each of the six actuators 600 and the seventh actuator based on the current position of the platform 200 and on the received target position of the platform 200 and commands one or more of the six actuators 600 and/or the seventh actuator to move accordingly.

The target position corresponds to a given location and orientation of the platform 200 in space.

One or several of the respective movement orders calculated by the control unit may be equal to zero, as not all of the six actuators 600 and/or seventh actuator necessarily need to be moved to change the position the base 100 from the current position to the target position.

The control unit may be configured to calculate the movement orders continuously and in real time and to send the movement orders continuously and in real time. Therefore, the position of the platform 200 may be adjusted continuously and in real time during the surgical procedure.

The control unit may be configured to send the movement orders simultaneously to the respective six actuators 600 and to the seventh actuator. Therefore, all the actuators which need to be actuated to move the platform 200 to the target position can be actuated simultaneously, which reduces the time necessary to bring the platform 200 to the target position.

### Surgical tool

The surgical apparatus may further comprise a surgical tool 500 in the form of an endoscope or a videoscope configured to be removably attached to the platform 200 by the fastening system.

The surgical tool 500 may be an endoscope. For example, the surgical tool 500 may be a bent endoscope, for example an L-shaped bent endoscope.

Alternatively, the surgical tool 500 may be a videoscope. For example, the videoscope may comprise an image sensor positioned at a second end of the videoscope opposite the first end attached to the platform 200. A videoscope is less bulky than an endoscope, as it only necessitates a wire to connect the base 100 to the image sensor.

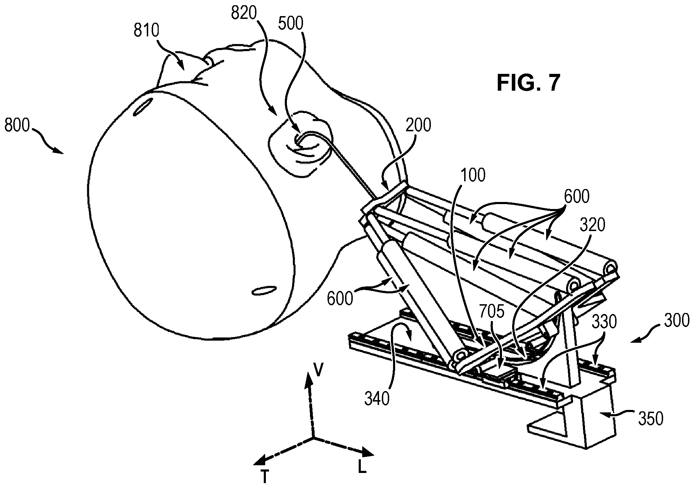

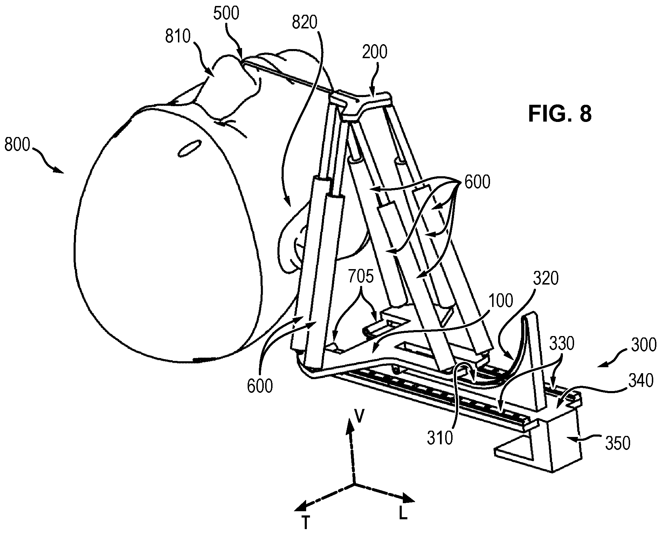

Such an endoscope or videoscope can be used in a wide variety of applications, more specifically in varied otorhinolaryngology procedures. For example, as illustrated in figure 7, the surgical tool 500 may be positioned at the ear 6120 of the patient 800, or alternatively, as illustrated in figure 8, the surgical tool 500 may be positioned at the nose 810 of the patient 800.

### Method for positioning a platform

A method for positioning a platform 200 of a device as disclosed above comprises the following steps:
- receiving a target position and orientation of the platform 200;
- calculating respective movement orders for the six actuators 600 and the seventh actuator in order to move the platform 200 to the target position and orientation; and
- sending the respective movement orders to the respective six actuators 600 and seventh actuator so as to correspondingly move the six actuators 600 and seventh actuator in order to reach the target position and orientation of the platform 200.

The method presents similar advantages to those disclosed above in relation to the device. More specifically, the method enables a precise and complex positioning of the platform 200, thus of a surgical tool 500 mounted on the platform 200, along the six degrees of freedom. Furthermore, the method can quickly and efficiently position the platform 200 in a wide range of positions, thus provides an extended work area and enabling the method to be used in a wide variety of surgical procedures.

The steps of the method may be performed by the control unit, as in a similar way as disclosed above in relation with the control unit. More specifically, the respective movement orders may be calculated continuously and in real time and the movement orders may be sent continuously and in real time.

The respective movement orders may be sent simultaneously to the respective six actuators 600 and to the seventh actuator.

Obviously, the present disclosure cannot be limited to the means and configuration described and illustrated herein, and it also extends to any equivalent means or configurations and to any technically operative combination of such means.

## Claims

1. A device for positioning a platform (200) according to six degrees of freedom, comprising:
- a base (100);
- a platform (200);
- six actuators (600), each comprising a first end pivotally attached to the base (100) and a second end pivotally attached to the platform (200), the six actuators (600) being configured to be individually or simultaneously actuated so as to rotate and translate the platform (200) relative to the base (100) according to the six degrees of freedom, wherein the device is **characterized in that** it further comprises:
- a seventh actuator configured to translate the base (100) along a longitudinal direction (L); and
- a guiding support (300) comprising a mounting system for a movable attachment of the base (100) in translation along the longitudinal direction (L) and in tilting around a tilt axis perpendicular to the longitudinal direction (L), wherein the guiding support (300) further comprises a mechanical tilting system cooperating with the base (100) and configured to tilt the base (100) around the tilt axis in response to a translation of the base (100) along the longitudinal direction (L) so that an actuation of the seventh actuator results in a combined translation of the base (100) along the longitudinal direction (L) and rotation of the base (100) around the tilt axis.

2. The device according to claim 1, wherein the mechanical tilting system is configured to maintain a tilt of the base (100) equal to zero when a stroke of the seventh actuator is below a predetermined threshold, and to increase a tilt of the base (100) when the stroke of the seventh actuator is above the predetermined threshold.

3. The device according to claim 1 or claim 2, wherein the mechanical tilting system comprises a rib having a flat portion (310) extending mainly along the longitudinal direction (L) and a curved portion (320), the mechanical tilting system further comprising an articulated joint mounting the base (100) on the rib.

4. The device according to claim 3, wherein the curved portion (320) has a fixed radius of curvature.

5. The device according to claim 3 or claim 4, wherein the curved portion (320) comprises a first end and a second end opposite to the first end along the longitudinal direction (L), wherein the first end of the curved portion (320) is a straight edge and the second end of the curved portion (320) is a straight edge, and wherein a first plane tangent to the curved portion (320) at a first point located on the first end is substantially perpendicular to a second plane tangent to the curved portion (320) at a second point located on the second end.

6. The device according to any one of claims 1 to 5, wherein the mounting system of the guiding support (300) comprises at least one linear longitudinal guide rail (330) and at least one sliding joint (335), each sliding joint (335) mounting the base (100) on a respective guide rail (330).

7. The device according to any one of claims 3 to 5 taken in combination with claim 6, wherein a central bore (110) is formed in the base (100) and wherein the rib or the guide rail (330) is configured to extend facing the central bore (110).

8. The device according to any one of claims 1 to 7, wherein the six actuators (600) comprise hydraulic and/or electric cylinders and/or wherein the seventh actuator is a hydraulic or an electric cylinder.

9. The device according to any one of claims 1 to 8, wherein the guiding support (300) comprises a fixing system (350) configured for removable attachment of the guiding support (300) to a component located in a surgical space.

10. The device according to any one of claims 1 to 9, wherein the platform (200) comprises a fastening system configured for removable attachment of a surgical tool (500) to the platform (200).

11. A surgical apparatus comprising a device according to any one of claims 1 to 10 and a control unit configured to:
- receive a target position and orientation of the platform (200);
- calculate respective movement orders for the six actuators (600) and the seventh actuator in order to move the platform (200) to the target position and orientation; and
- send the respective movement orders to the respective six actuators (600) and seventh actuator so as to correspondingly move the six actuators (600) and seventh actuator in order to reach the target position and orientation of the platform (200).

12. The surgical apparatus according to claim 11, wherein the device is according to claim 10 and the surgical apparatus further comprises a surgical tool (500) in the form of an endoscope or a videoscope configured to be removably attached to the platform (200) by the fastening system.

13. The surgical apparatus according to claim 11 or claim 12, wherein the control unit is configured to calculate the movement orders continuously and in real time and to send the movement orders continuously and in real time, simultaneously to the six actuators (600) and to the seventh actuator.

14. A method for positioning a platform (200) of a device according to any one of claims 1 to 10, comprising the following steps:
- receiving a target position and orientation of the platform (200);
- calculating respective movement orders for the six actuators (600) and the seventh actuator in order to move the platform (200) to the target position and orientation; and
- sending the respective movement orders to the respective six actuators (600) and seventh actuator so as to correspondingly move the six actuators (600) and seventh actuator in order to reach the target position and orientation of the platform (200).
